# EUROPEAN PATENT SPECIFICATION

(11) **EP 2 334 668 B1**
(45) Date of publication and mention of the grant of the patent: **02.04.2014**
(21) Application number: 09782721.6
(22) Date of filing: 07.09.2009
(51) Int. Cl.: C07D 405/14, A61K 31/41, A61P 9/12

(54) **PROCESS FOR PREPARING OLMESARTAN MEDOXOMIL INTERMEDIATE**
VERFAHREN ZUR HERSTELLUNG EINES OLMESARTANMEDOXOMILZWISCHENPRODUKTS
PROCÉDÉ DE PRÉPARATION D'UN INTERMÉDIAIRE D'OLMÉSARTAN MÉDOXOMIL

(30) Priority: 05.09.2008 SI 200800208
(43) Date of publication of application: 22.06.2011
(73) Proprietor: Krka Tovarna Zdravil, D.D., Novo Mesto, 8501 Novo Mesto (SI)
(72) Inventor: ZUPANCIC, Silvo, 8000 Novo Mesto (SI)
(74) Representative: HOFFMANN EITLE
(86) International application number: PCT/EP2009/061583
(87) International publication number: WO 2010/026255

(56) References cited:
- EP-A2- 1 916 246
- WO-A1-2007/048361
- WO-A2-2007/047838
- US-A- 5 616 599

## Description

### Field of the invention

The present invention is directed to a process for preparing an olmesartan medoxomil intermediate of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate with purity greater than 98% and further converting such an intermediate to olmesartan medoxomil. The process according to the invention are cost and time effective and produce olmesartan and/or pharmaceutically acceptable salts thereof with high yield and quality.

### Background of the invention

Olmesartan medoxomil with its chemically name (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate and formula 1 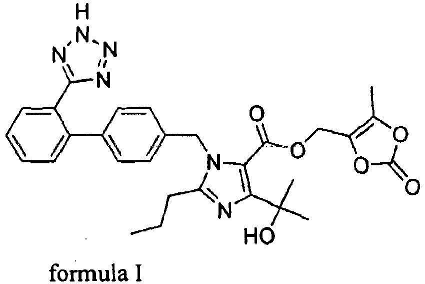 belongs to the class of medicines called angiotensin II receptor antagonists to treat high blood pressure and related diseases and conditions. It can be used alone or in combination with another pharmaceutically active compound, e.g. hydrochlorothiazide, amlodipine and similar.

Olmesartan and pharmaceutically acceptable salts thereof has been first described in EP 0 503 785 B1. In EP 0 503 785 B1 process for the preparation of olmesartan medoxomil is disclosed involving inter alia reacting (5-methyl-2-oxo-1,3-dioxolen-4-yl)methyl-4-(1-hydroxy- 1-me- thylethyl)-2-propylimidazole-5-carboxylate and 4-[2-trityltetrazol-5-yl)phenyl]benzyl bromide in N,N-dimethyacetamide in the presence of potassium carbonate, or reacting ethyl-4- (1 -hydroxy- I-methylethyl)-2-propylimidazole-5-carboxylate and 4-[2-trityltetrazol-5-yl)phenyl]benzyl bromide in N,N-dimethylformamide in the presence of sodium hydride. Common to all the processes disclosed is that the alkylated product is subjected to a column chromatography in order to obtain an acceptable purity. For the preparation of an ester, the product obtained is described to be hydrolized by means of an alkali metal hydroxide, the salt is isolated and further esterified. In the last step, the trityl protection group is removed by reacting the tritylolmesartan medoxomil ester in acetic acid.

WO 2004085428 discloses the process for preparing olmesartan medoxomil wherein 4,4-dimethyl-2propyl-1-(4-[2-(triphenylmethyltetrazole-5-yl)phenyl]phenyl)methyl4,6-dihydrofuran[3,4d]imidazole-6-one is ring-opened, the resulted 4-(1-hydroxy-1-methylethyl)-2-propyl-1-(4-[2-(triphenylmethyltetrazole-5-yl)phenyl]phenyl)methylimidazole-5-carboxylic acid is subsequently condensed with 4-bromo(or chloro)methyl-5-methyl-2-oxy-1,3-dioxyheterocyclopentene under the action of alkali and after removing the triphenylmethyl protective group olmesartan medoxomil is obtained.

WO 2006/029056 discloses a process for preparing olmesartan medoxomil comprising contacting tritylolmesartan medoxomil with an acid in a water miscible organic solvent to obtain a solution of olmesartan medoxomil and a precipitate of triphenyl carbinol; separating the precipitate of triphenylcarbinol from the solution of olmesartan medoxomil; contacting the solution of olmesartan medoxomil with a base to obtain a precipitate of olmesartan medoxomil; and recovering olmesartan medoxomil.

WO 2006/073518 discloses a process for preparing olmesartan medoxomil comprising dissolving tritylolmesartan medoxomil in a mixture of an organic solvent and water to form a solution with a pH of at least 2.5 and then heating the solution to obtain olmesartan medoxomil.

WO 2006/073519 provides a process for preparing olmesartan medoxomil containing less than about 0.1% of one or more of impurities 4-(1-methoxy-1-methylethyl)-2-propyl-1-[2'-(1H-tetrazole-5-yl)biphenyl-4-ylmethyl]imidazole-5-carboxylic acid 5-methyl-2-oxo-1,3-dioxol-4-ylmethyl ester, 4-(1-hydroxy-1-methylethyl)-2-propyl-1-[2'-(1H-tetrazole-5-yl)biphenyl-4-yl-methyl]imidazole-5-carboxylic acid 5-chloromethyl-2-oxo-1,3-dioxol-4-ylmethyl ester and 4-(1-methylethylene)-2-propyl-1-[2'-(1H-tetrazole-5-yl)biphenyl-4-ylmethyl]imidazole-5-carboxylic acid 5-chloromethyl-2-oxo-1,3-dioxol-4-yl methyl ester, wherein the sample of tritylolmesartan medoxomil further used in the synthesis of olmesartan medoxomil is selected from the samples in which the amount of one or more of the measured impurities is less than about 0.1%.

WO 2007/017135 discloses a process for the preparation of olmesartan medoxomil which comprises alkylating ethyl 4-(1-hydroxy-1-methylethyl)-2-propylimidazole-5-carboxylate with 4-[2-(trityltetrazol-5-yl)phenyl]-benzyl bromide or 4'-bromomethylbiphenyl-2-carbonitrile in an organic solvent and in the presence of a base, wherein acetonitrile is used as the reaction and the crystallization solvent to obtain tritylolmesartan which is further converted to tritylolmesartan medoxomil and olmesartan medoxomil as an one-pot reaction.

WO 2007/048361 disclosing the process of deprotection of tritylolmesartan medoxomil by performing the reaction with water in the presence of a solvent which is partially or completely miscible with water, such as for example acetone, acetonitrile.

WO 2007/148344 provides a process for the preparation of olmesartan medoxomil by condensing the ethyl 4-(1-hydroxy-1-methylethyl)-2-propylimidazole-5-carboxylate with 4-[2-(trityl tetrazol-5-yl) phenyl] benzyl bromide to obtain ethyl 4-(1-hydroxy-1-methyl ethyl)-2-propyl-1-(4-[2-(trityltetrazol-5-yl)phenyl]phenyl)methylimidazole-5-carboxylate and then hydrolyzing ethyl 4-(1-hydroxy-1-methyl ethyl)-2-propyl-1-(4-[2-(trityl tetrazol-5-yl) phenyl]phenyl)methyl imidazole-5-carboxylate to obtain tritylolmesartan dihydrate followed by reacting with 4-chloromethyl-5-methyl-2-oxo-1,3-dioxolene to obtain tritylolmesartan medoxomil and then deprotecting tritylolmesartan medoxomil to obtain olmesartan medoxomil.

According to EP 1916246 olmesartan medoxomil of high purity is prepared by a process wherein ethyl-4-(1-hydroxy-1-methylethyl)-2-propylimidazole-5-carboxylate is reacted with N-(triphenylmethyl)-5-[4'-(bromomethyl)biphenyl-2-yl]tetrazole in an organic solvent in the presence of a base and a phase transfer catalyst in a non-aqueous system to give ethyl-4-(1-hydroxy-1-methylethyl)-2-propyl-1-[[2'-[2-(triphenylmethyl)-2H-tetrazol-5yl]biphenyl-4-yl]methyl]imidazole-5-carboxylate, which is further crystallized using isopropyl alcohol followed by purification from methyl ethyl ketone to provide substantially pure [HPLC purity 99.3 to 99.7 %] olmesartan medoxomil.

WO 2008/043996 discloses a process for the preparation of tritylolmesartan comprising condensing 4-(1-hydroxy-1-methylethyl)-2-propyl-imidazol-5-carboxylic acid alkyl ester with trityl biphenyl bromide in the presence of a polar aprotic solvent and a base, deesterifying with a base; and reacting the obtained product with 4-halomethyl-5-methyl-2-oxo-1,3-dioxolene to obtain tritylolmesartan medoxomil that may be deprotected to produce olmesartan medoxomil.

In addition to the above-discussed preparation processes of olmesartan and pharmaceutically acceptable salts thereof, there still is a need for a yet further improved synthetic route to olmesartan medoxomil.

Therefore, the object of the present invention is to provide new methods for the production of an olmesartan medoxomil intermediate of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate which methods are suitable for use on an industrial scale and which are economical, i.e. both cost and time effective and allow for the production of intermediates that can be converted into olmesartan medoxomil and/or salts thereof with high quality and high yield.

### Summary of the invention

The first aspect of the present invention relates to the process for the preparation of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate (tritylolmesartan medoxomil) comprising the steps of:
- esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid with a 4-halomethyl-2-oxo-1,3-dioxolene derivative in the presence of an inorganic base and an iodide and optionally in an organic solvent wherein halo means Cl or Br,
- providing a solution tritylolmesartan medoxomil in organic solvent selected from the group consisting of ethyl acetate, isopropyl acetate, propyl acetate, butyl acetate, isobutyl acetate, *tert*-butyl acetate; toluene, cyclohexane, hexane, heptane, diethyl ether, *tert*-butylmethyl ether and mixtures thereof,
- optionally concentrating the solution, and
- addition of another solvent selected from group consisting of acetone, acetonitrile, isopropanol and ethanol and
- isolating solid tritylolmesartan medoxomil.

According to the second aspect of the present invention, the tritylolmesartan medoxomil obtained in the process of the first aspect is converted to olmesartan medoxomil by the subsequent deprotection of the trityl protection group.

According to one specific aspect of the present invention pure solid tritylolmesartan medoxomil having purity greater than 98%, preferably greater than 99% is obtained by the process according to the present invention and then converted to olmesartan medoxomil.

According to one specific aspect of the present invention tritylolmesartan medoxomil specified by an X-ray powder diffraction pattern exhibiting peaks at the following diffraction angles: 10.2 and 21.7 20 is obtained by the process according to the present invention and then converted to olmesartan medoxomil.

According to one specific aspect of the present invention tritylolmesartan medoxomil specified by an X-ray powder diffraction pattern exhibiting peaks at the following diffraction angles: 3.0, 10.2, 12.1, 15.6, 18.4, 21.7 and 24.3 2Θ is obtained by the process according to the present invention and then converted to olmesartan medoxomil.

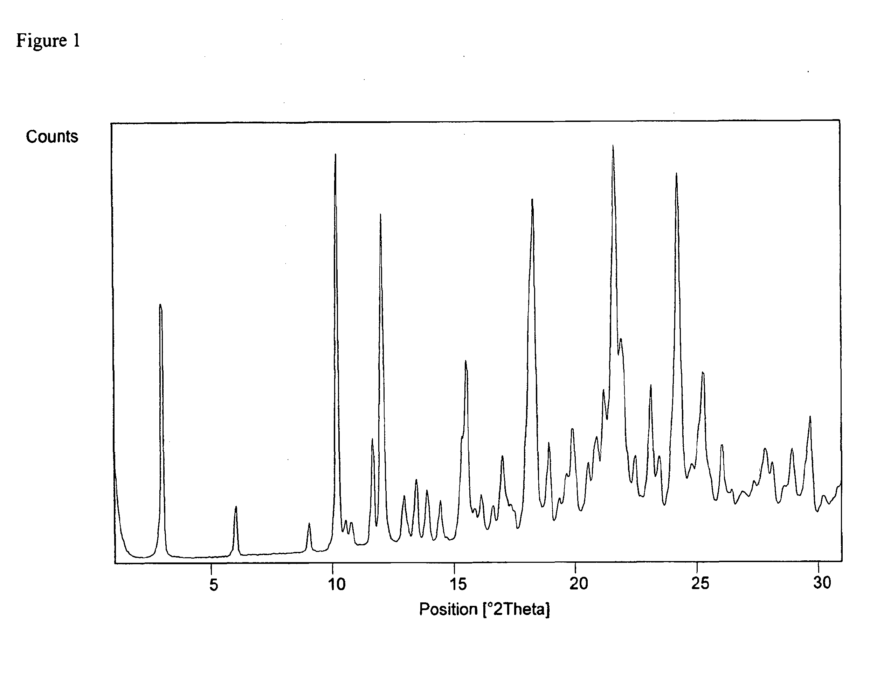

According to one specific aspect of the present invention tritylolmesartan medoxomil having an X-ray powder diffraction pattern as shown in Figure 1 is obtained by the process according to the present invention and then converted to olmesartan medoxomil.

Another specific aspect of the present invention is a process for preparing (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate wherein an iodide selected from the group consisting of KI, NaI, LiI, preferably KI, is added in the step of esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid and wherein the isolation of the obtained compound is performed in an organic solvent as outlined above with respect to the first aspect of the invention.

### Brief description of the figures

Figure 1: Powder X-ray diffraction pattern of tritylolmesartan medoxomil

### Detailed description of the invention

The object of the present invention is to provide novel time- and cost-effective process for the preparation of an olmesartan intermediate of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate called tritylolmesartan medoxomil with purity greater than 98%.

Therefore, the first aspect of the present invention relates to the process for the preparation of tritylolmesartan medoxomil comprising the steps of:
- esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid or salt thereof with a 4-halomethyl-2-oxo-1,3-dioxolene derivative in the presence of an inorganic base and an iodide and optionally in an organic solvent wherein halo means Cl or Br,
- providing a solution tritylolmesartan medoxomil in organic solvent selected from the group consisting of ethyl acetate, isopropyl acetate, propyl acetate, butyl acetate, isobutyl acetate, *tert*-butyl acetate; toluene, cyclohexane, hexane, heptane, diethyl ether, *tert*-butylmethyl ether and mixtures thereof,
- optionally concentrating the solution, and
   addition of another solvent selected from group consisting of acetone, acetonitrile, isopropanol and ethanol and- isolating solid tritylolmesartan medoxomil. The use of ethyl acetate, acetonitrile or a combination of ethyl acetate and acetonitrile is preferred.

The starting compound 4-(2-hydroxypropan-2-yl)-2-propyl-1-((2'-(1-trityl-1H-tetrazol-5-yl)biphenyl-4-yl)methyl)-1H-imidazole-5-carboxylic acid can be prepared by any method known from the prior art as for example from WO 2004/085428, WO 2007/017135 and WO 2007148344. The other starting material is either 4-(chloromethyl)-5-methyl-2-oxo-1,3-dioxolene (4-(chloromethyl)-5-methyl-1,3-dioxol-2-one) or 4-(bromomethyl)-5-methyl-2-oxo-1,3-dioxolene (4-(bromomethyl)-5-methyl-1,3-dioxol-2-one), the two compounds being collectively referred to as 4-halomethyl-2-oxo-1,3-dioxolene derivative with halo representing either chloro or bromo. The chloro derivative is preferred.

Alternatively, the starting compound 4-(2-hydroxypropan-2-yl)-2-propyl-1-((2'-(1-trityl-1H-tetrazol-5-yl)biphenyl-4-yl)methyl)-1H-imidazole-5-carboxylic acid can be prepared without isolation from ethyl 4-(2-hydroxypropan-2-yl)-2-propyl-1-((2'-(1-trityl-1H-tetrazol-5-yl)biphenyl-4-yl)methyl)-1H-imidazole-5-carboxylate by the following steps:
- suspending of ethyl 4-(2-hydroxypropan-2-yl)-2-propyl-1-((2'-(1-trityl-1H-tetrazol-5-yl)biphenyl-4-yl)methyl)-1H-imidazole-5-carboxylate in an organic solvent that can be selected from, but not limited to, dimethylformamide (DMF), N,N-dimethylacetamide (DMA), dimethyl sulfoxide (DMSO), acetonitrile and any mixtures thereof, preferably DMF, DMA, more preferably DMA.
- addition of an inorganic base such as for example KOH, NaOH, LiOH,
- performing the hydrolysis at room temperature for 1 to 24h, preferably for 1 to 5h.

The starting compound ethyl 4-(2-hydroxypropan-2-yl)-2-propyl-1-((2'-(1-trityl-1H-tetrazol-5-yl)biphenyl-4-yl)methyl)-1H-imidazole-5-carboxylate can be prepared by any method known from the prior art as for example from EP 0 503 785 A1, JP 06087833 and WO 2007/017135.

The term 4-(2-hydroxypropan-2-yl)-2-propyl-1-((2'-(1-trityl-1H-tetrazol-5-yl)biphenyl-4-yl)methyl)-1H-imidazole-5-carboxylic acid, as it is used in the context of the present text, may be understood to include carboxylic acid salts thereof (which may for instance be formed by the above-mentioned hydrolysis procedure).

Esterification step of the process for the preparation of tritylolmesartan medoxomil according to the present invention comprises the following steps:
- optionally providing the solution of 4-(2-hydroxypropan-2-yl)-2-propyl-1-((2'-(1-trityl-1H-tetrazol-5-yl)biphenyl-4-yl)methyl)-1H-imidazole-5-carboxylic acid in an organic solvent that can be selected from, but not limited to, dimethyl formamide (DMF), N,N-dimethylacetamide (DMA), dimethyl sulfoxide (DMSO), acetonitrile and any mixtures thereof, preferably DMF, DMA, more preferably DMA,
- addition of an inorganic base such as for example hydroxydes such as for example KOH, NaOH, LiOH, carbonates such as for example Na₂CO₃, K₂CO₃, phosphates such as for example Na₃PO₄, preferably Na₂CO₃, K₂CO₃, more preferably K₂CO₃,
- addition of an iodide preferably selected from the group consisting of KI, NaI, LiI, more preferably KI,
- addition of 4-halomethyl-2-oxo-1,3-dioxolene derivative wherein halo means Cl or Br,
- performing esterification preferably at elevated temperature as for example between 20 and 60°C preferably for 1 to 24h, more preferably for 1 to 5h.

Whilst it is possible to perform these steps in virtually any relative order including simultaneously effecting two or more of the specified steps (the only limitation being the fact that the esterification cannot be performed unless all reagents are present), the process steps according to one aspect of the invention are carried out in the specified order.

Crystallization step for the preparation of pure tritylolmesartan medoxomil according to the present invention comprises the following step:
- providing the solution tritylolmesartan medoxomil in organic solvent selected from group of ethyl acetate, isopropyl acetate, propyl acetate, butyl acetate, isobutyl acetate, *tert*-butyl acetate; toluene, cyclohexane, hexane, heptane, diethyl ether and *tert*-butylmethyl ether
- optionally concentrating the solution
- addition of another solvent selected from group of acetone, acetonitrile, isopropanol and ethanol.

Crystals of tritylolmesartan medoxomil precipitate after the addition of the other solvent. Said crystals may then be isolated by means of filtration or centrifugation, optionally be washed with e.g. one of the above solvents, preferably acetonitrile, and then optionally be dried in the usual manner. This crystallization process allows to obtain the specific polymorph of tritylolmesartan medoxomil characterized below.

The tritylolmesartan medoxomil prepared according to the process of the present invention has purity greater than 98%, preferably greater than 99%. The advantage of said pure solid tritylolmesartan medoxomil is that it can be easily converted by already known procedures to olmesartan medoxomil without additional purification neither of the used tritylolmesartan medoxomil neither of the obtained olmesartan medoxomil and without preceding selection of the samples that contain a predefined low amount of impurities.

Therefore, one specific aspect of the present invention relates to a process wherein pure solid tritylolmesartan medoxomil having purity greater than 98%, preferably greater than 99%, the most preferably greater than 99.5 % with impurities less than 0.1 % is obtained and then converted to olmesartan medoxomil.

A further specific aspect of the present invention relates to a process wherein tritylolmesartan medoxomil specified by an X-ray powder diffraction pattern exhibiting peaks at the following diffraction angles: 10.2 and 21.7 2Θ is obtained and then converted to olmesartan medoxomil.

A further aspect of the present invention relates a process wherein tritylolmesartan medoxomil specified by an X-ray powder diffraction pattern exhibiting peaks at the following diffraction angles: 3.0, 10.2, 12.1, 15.6, 18.4, 21.7 and 24.3 20 is obtained and then converted to olmesartan medoxomil.

| No. | Pos. [°2Th.] | d-spacing [A] | Rel. Int. [%] |
|---|---|---|---|
| 1 | 3.0 | 29.25 | 62 |
| 2 | 10.2 | 8.64 | 100 |
| 3 | 12.1 | 7.33 | 85 |
| 4 | 15.6 | 5.69 | 47 |
| 5 | 18.4 | 4.83 | 86 |
| 6 | 21.7 | 4.10 | 100 |
| 7 | 24.3 | 3.66 | 93 |

According to another aspect the tritylolmesartan medoxomil obtained in accordance with the present invention has an X-ray powder diffraction pattern as shown in Figure 1.

We surprisingly found out that the purity of tritylolmesartan medoxomil, that is an important and key intermediate in the synthesis of olmesartan medoxomil, is substantially influenced by the following key factors:
- addition of an iodide in the step of esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid wherein an iodide is preferably selected from the group consisting of KI, NaI, LiI, preferably KI,
- isolating of solid tritylolmesartan medoxomil from an organic solvent as specified in appended claim 1.

Hence, in one aspect, the present invention relates to a process for preparing (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate wherein an iodide selected from the group consisting of KI, NaI, LiI, preferably KI, is added in the step of esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid and wherein the isolation of the obtained compound is performed as specified in appended claim 1.

Moreover, the process for the preparation of tritylolmesartan medoxomil from ethyl 4-(2-hydroxypropan-2-yl)-2-propyl-1-((2'-(1-trityl-1H-tetrazol-5-yl)biphenyl-4-yl)methyl)-1H-imidazole-5-carboxylate according to the present invention gives high yield, particularly more than 80%, preferably more than 90%.

Another aspect of the present invention is the conversion of pure solid tritylolmesartan medoxomil prepared by the process of the present invention to olmesartan medoxomil by the subsequent deprotection of the trityl protection group. This process can be performed by any method known from the prior art as for example from EP 0 503 785 A1, WO 2004/085428, 2006/029056, WO 2006/073518, WO 2007/017135, WO 2007/048361, WO 2007/148344, WO 2008/043996.

Olmesartan medoxomil particles prepared according to the present invention may be characterised by particle size or by specific surface area.

The mean particle size of Olmesartan medoxomil particles is between 1 and 150 microns, preferably between 5 and 120 microns, more preferably between 10-100 microns as determined by laser light diffraction instrument Malvern Mastersizer 2000 with purified water used as the dispersion medium (A=1).

The specific surface area of olmesartan medoxomil particles is preferably between 0.1 and 7 sq.m/g, more preferably between 0.3 and 4 sq.m/g. Specific surface area was measured on Tristar 3000 instrument using 6-point BET calculation. The samples were measured after 2h of vacuum degassing.

Another aspect of the present invention is a process for manufacturing a pharmaceutical composition comprising the use of olmesartan medoxomil prepared from tritylolmesartan medoxomil prepared by the process according to the present invention. Said pharmaceutical composition may be prepared by any known technological process (as for example direct compression, dry or wet granulation and similar) and may contain any pharmaceutically acceptable ingredients such as for example binders, surfactants, diluents, disintegrants, lubricants and optionally coating layer. Said pharmaceutical composition may further contain one or more other active substance selected from the group of antihypertensives, diuretics, lipid regulators or antidiabetics. Antihypertensive can be selected from the group of angiotensive converting enzyme (ACE) inhibitors, angiotensin receptor blockers (ARBs), calcium channel blockers (CCBs) and antiadrenergics. Lipid regulators can for example be HMG CoA reductase inhibitors. Preferably the other active substance is selected from the group consisting of captopril, enalapril, cilazapril, lisinopril, trandolapril, ramipril, fosinopril, perindopril, amlodipine, diltiazem, felodipine, nifedipine, nitrendipine, verapamil, acebutol, atenolol, betaxolol, bisoprolol, metoprolol, carvedilol, lovastatin, simvastatin, pravastatin, atorvastatin, fluvastatin, rosuvastatin, indapamide, hydrochlorothiazide, sulfonyl urea, metglinides such as nateglinide or repaglinide, thiazolidinediones such as pioglitazone or rosiglitazone, α-glucosidase inhibitors, incretin mimetics, biguanides such as metformin hydrochloride and pharmaceutically acceptable salts thereof.

The pharmaceutical composition according to one aspect of the invention, comprising olmesartan medoxomil prepared from tritylolmesartan medoxomil prepared by the process according to the present invention and one or more other active substance such as for example a diuretic, preferably hydrochlorothiazide, may be prepared by any known technological processes such as direct compression, wet granulation (with water or organic solvents, such as for example methanol, or any mixtures thereof), dry granulation or lyophilization. Preferably, direct compression process may be used.

The direct compression process may be performed in the way that
(a) at least one active ingredient is added to the mixture of excipients and compressed, or
(b) at least one active ingredient is mixed together with excipients and compressed.
Solid dosage form (e.g. tablet cores) can be optionally coated.

The direct compression process is preferably performed if the pharmaceutical composition contains a low percentage of active ingredient in total weight of tablet. The term "by percentage" is meant to indicate % by weight of active ingredient in total weight of tablet. The term "low percentage of active ingredient" is meant to indicate less than 20% by weight of active ingredient in total weight of tablet.

Suitable mixing device in the direct compression or optionally, wet granulation as described above is conventional equipment used for mixing of active ingredients, excipients or combination of active ingredient(s) and excipients. Conventional equipment is motionless (passive) mixers, fluidized beds, diffusion, biconic diffusion, biconic, turbula, cubic, planetary, Y-, V-shaped or high-shear mixer, drum etc. In the case of wet granulation as described below, the equipment is chosen from standard equipment for drying, i.e. fluid-bed dryer, plates, etc.

When hydrochlorothiazide is used as other active substance the mean particle size of its particles is less than 110 microns, preferably less than 90 microns, more preferably between 20 and 80 microns as determined by laser light diffraction instrument Malvern Mastersizer 2000 with vegetable oil used as the dispersion medium (A=1).

Excipients may optionally be processed by wet granulation, using either water or organic solvent or any mixture thereof as granulating liquid. By processing of excipients by wet granulation, it means homogenisation of excipients and addition of granulating liquid to the mixture thereof. Granulating liquid can optionally contain binder or binders, either individual or any mixtures thereof.

Furthermore, when wet granulation is used, olmesartan medoxomil can be included either into the intragranular phase of a granulate and/or into the extragranular phase, i.e. as an additive to the granulate. Furthermore, one or more other active substance diuretic can be included either into the intragranular phase of a granulate and/or into the extragranular phase, i.e. as an additive to the granulate. The granulate may be, for example, a granulate for tablet compression or a granulate for filling capsules. Moreover, one or more other active substance diuretic can be included in coating layer provided on a core. The core may be individual granules of a granulate, or a core obtained by compressing a granulate or powderly material. These modes of inclusion can be used in combination with each other.

In another aspect of the invention, using wet granulation, olmesartan medoxomil and one or more other active substance diuretic may be partially included into intragranular and extragranular phase, i.e. they may be divided between intragranular and extragranular phase in any proportions.

The pharmaceutical composition according to the present invention may be, for example, immediate release dosage form, a fast melt dosage form, controlled release dosage form, lyophilized dosage form, delayed release dosage form, extended release dosage form, pulsatile release dosage form, mixed immediate release and controlled release dosage form, or a combination thereof. The pharmaceutical composition is preferably tablet formulation, which can be optionally coated. The pharmaceutical composition is preferably an immediate release dosage form offering advantages regarding the bioavailability of the active compound.

If an immediate release dosage form is chosen, it will be clear for the skilled person that the amount of release controlling agent or agents, either individual or mixture thereof to be used in forming the outer portion will be determined based on various parameters such as the desired delivery properties, including the amount of active ingredient or substance to be delivered, the active ingredient or substance release rate desired, and the size of the micro matrix particles.

The pharmaceutical composition manufactured according to the present invention may consist of:
- 1-99%, preferably 5-50%, more preferably 5-15% by weight of olmesartan medoxomil prepared from tritylolmesartan medoxomil prepared by the process according to the present invention,
- optionally 1-99%, preferably 1-50%, more preferably 1-15% by weight of one or more other active substance, preferably a diuretic,
- 1-99%, preferably 20-99%, more preferably 50-99% by weight of diluent,
- 1-90%, preferably 1-50% by weight of binder,
- 1-50%, preferably 2-40% by weight of disintegrant or superdisintegrant,
- 0.1-10%, preferably 0.1-5% by weight of lubricant, and
- optionally, 0.1-10%, preferably 0.1-5% by weight of surfactant, and
- optionally, 0.1 to 10% film coating layer.

The excipients present in the pharmaceutical composition manufactured according to the present invention may comprise diluents such as for example microcrystalline cellulose, powdered cellulose, lactose (anhydrous or monohydrate), compressible sugar, fructose, dextrates, other sugars such as mannitol, sorbitol, maltitol, xylitol, lactitol, isomalt or other sugars such as saccharose, raffinose, trehalose, fructose, or mixture thereof, siliconised microcrystalline cellulose, calcium hydrogen phosphate, calcium carbonate, calcium lactate or combined diluents. Preferably, the excipients include at least one diluent selected from microcrystalline cellulose and lactose monohydrate.

The pharmaceutical composition manufactured according to the present invention may also comprise binders, such as for example povidone, microcrystalline cellulose, hydroxyethylcellulose, hydroxypropylcellulose, low-substituted hydroxypropylcellulose (comprising from 5 to 16% by weight of hydroxypropyl groups), hydroxypropylmethylcellulose or other cellulose ethers, starch, pregelatinised starch or polymethacrylate or mixture of binders. It is preferred that excipients include at least one binder selected from microcrystalline cellulose and low-substituted hydroxypropylcellulose.

Further, disintegrants and/or superdisintegrants may also be present such as for example starch (e.g. maize starch, potato starch), modified starches (sodium starch glycolate), modified cellulose (croscarmellose, i.e. cross-linked carboxymethlycellulose sodium), cross-linked polyvinylpyrrolidone (crospovidone), microcrystalline cellulose, carboxymethylcellulose sodium, Amberlite^{®}, alginic acid, sodium alginate, guar gum, gellan gum, and any mixtures thereof., Xanthan SM^{®}. If used as a disintegrant, microcrystalline cellulose is preferably used in an amount of 5 to 15% by weight. It is preferred that excipients include at least one disintegrant or superdisintegrant selected from croscarmellose, crospovidone and microcrystalline cellulose.

Further, lubricants may also be present as excipients, such as stearic acid, magnesium stearate, magnesium palmitate, magnesium oleate, calcium stearate, sodium laurylsulphate, hydrogenated vegetable oil, hydrogenated castor oil, sodium stearyl fumarate, talc, macrogols, and any mixtures thereof. It is preferred that the excipients include at least one lubricant selected from magnesium stearate, talc and macrogols.

Suitable non-ionic surfactants may for example be selected from the group consisting of alkylglucosides, alkylmalto- sides, alkylthioglukosides, lauryl macrogolglycerides, polyoxyethylene alkylphenols, poly- oxyethylene alkylethers, polyethylene glycol fatty acid esters, polyethylene glycol glycerol fatty acid esters, polyoxyethylene sorbitan fatty acid esters, polyoxyethylene-polyoxypropylene block copolymers, polyglyceryl fatty acid esters, polyoxyethylene glycerides, polyoxyethylene vegetable oils, polyoxyethylene hydrogenated vegetable oils, sterols, and mixtures thereof. Preferred non-ionic surfactants are polyoxyethylene sorbitan fatty acid esters, which are sold under the trade names Polysorbate or Tween.

Suitable ionic surfactants may for example be selected from group of fatty acid salts, bile salts, phospho- lipides, phosphoric acid esters, carboxylates, sulphates, sulphonates and mixture thereof. A preferred ionic surfactant is sodium laurylsulphate.

Excipents may have multiple functions, i.e. one excipient may be diluent and additionally binder, binder and disintegrant etc.

Optionally, the tablet cores may for example be coated with conventional materials used for film coating. Film coating formulations may contain the following components: polymer(s), plasticizer(s), colourant(s)/opacifier(s), vehicle(s). In film coating suspension it is possible to use minor quantities of flavours, surfactants and waxes. The polymers used in film coating are preferably either cellulose derivatives, such as the cellulose ethers, or acrylic polymers and co-polymers. Also suitable are high molecular weight polyethylene glycols, polyvinyl pyrrolidone, polyvinyl alcohol and waxy materials.

Cellulose ethers may for example be selected from the group consisting of hydroxyethylcellulose, hydroxypropylcellulose, hydroxypropylmethylcellulose, methylcellulose. Acrylic polymers comprise a group of synthetic polymers with diverse functionalities. Some of them can be further modified to enhance swelling and permeability by the incorporation of materials such as water soluble cellulose ethers and starches in order to ensure complete disintegration /dissolution of the film.

Some suitably used plasticizers may for example be selected from the group consisting of polyols (glycerol, propylene glycol, macrogols), organic esters (phthalate esters, dibutyl sebacetate, citrate esters, triacetin), and oils/glycerides (castor oil, acetylated monoglycerides, fractionated coconut oil).

Colourants/opacifiers may for example be selected from organic dyes and their lakes, inorganic colours, natural colours.

Different materials from each group can be combined in defined ratio. Film coating suspensions can be used as ready-to-make preparations that are available on the market.

Film coating dispersion can be prepared by using solvents such as water, alcohols, ketones, esters, chlorinated hydrocarbons and any mixtures thereof, preferably water.

The following composition of coating suspension (calculated on dry material) comprising:
- 1-99% by weight of polymer, preferably 1-95% of polymer,
- 1-50% by weight of plasticizer, preferably 1-40% of plasticizer,
- 0.1-20% of colourant/opacifier, preferably 0,1-10% of colourant/opacifier
is particularly preferred.

The immediate release dosage form may also include material that improves the processing of the release controlling agents. Such materials are generally referred to as plasticizers. Preferred plasticizers may for example include acetylated monoglycerides, butyl phthalyl butyl glycolate, dibutyl tartrate, diethyl phthalate, dimethyl phthalate, ethyl phthalyl ethyl glycolate, glycerin, ethylene glycol, propylene glycol, triethyl citrate, triacetin, tripropinoin, diacetin, dibutyl phthalate, acetyl monoglyceride, polyethylene glycols, castor oil, triethyl citrate, polyhydric alcohols, acetate esters, glycerol triacetate, acetyl triethyl citrate, dibenzyl phthalate, dihexyl phthalate, butyl octyl phthalate, diisononyl phthalate, butyl octyl phthalate, dioctyl azelate, epoxidised tallate, triisoctyl trimellitate, diethylhexyl phthalate, di-n-octyl phthalate, dioctyl phthalate, di-i-decyl phthalate, di-n-undecyl phthalate, di-n-tridecyl phthalate, tri-2-ethyl- hexyl trimellitate, di-2-ethylexyl adipate, di-2-ethylhexyl sebacate, di-2-ethylhexyl azelate, dibutyl sebacate, glyceryl monocaprylate, glycerol distearate, glyceryl monocaprate, and any mixtures thereof.

The present invention is illustrated by the following examples without limiting it thereto.

Melting points were taken on a Koffler melting point apparatus and IR spectra were taken on a Paragon 100 Perkin-Elmer FT-IR spectrometer.

X-ray powder diffraction patterns were obtained by a Phillips PW3040/60 X'Pert PRO diffractometer using CUK_{α} radiation of 1,541874 A.

### HPLC Method:

### HPLC area percent method.

| | | |
|---|---|---|
| **Column:** | Zorbax Eclipse XDB C-18, 1.8 µm, 50 x 4.6 mm | |
| | | |
| **Mobile phase:** | solvent A: | 0.01M sodium dihydrogen phosphate, pH 2.8 |
| | solvent B: | acetonitrile |

### Gradient:

| Time (min) | %A | %B |
|---|---|---|
| 0 | 85 | 15 |
| 1 | 85 | 15 |
| 8 | 40 | 60 |
| 25 | 20 | 80 |
| 27 | 15 | 85 |
| 28 | 84 | 15 |

| | |
|---|---|
| **Post time:** | 3 min |
| **Temperature of column:** | 25°C |
| **Flow:** | 1.0 ml/min |
| **Detection:** | UV, 225 nm |
| **Injection:** | 10 µl |

### Example 1: Tritylolmesartan medoxomil; ((5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate) from ethyl 4-(2-hydroxypropan-2-yl)-2-propyl-1-((2'-(1-trityl-1H-tetrazol-5-yl)biphenyl-4-yl)methyl)-1H-imidazole-5-carboxylate

130 ml of *N*,*N*-dimethylacetamide, 14.0 g (20 mmol) of ethyl 4-(2-hydroxypropan-2-yl)-2-propyl-1-((2'-(1-trityl-1H-tetrazol-5-yl)biphenyl-4-yl)methyl)-1H-imidazole-5-carboxylate and 2.2 g (16 mmol) of KOH were charged into reaction vessel at room temperature under inert atmosphere. The mixture was stirred at room temperature for 2 h, and then the sample of reaction mixture was analysed (HPLC; starting material 0.2 %, hydrolysed starting material 98.11 %). Then 3.0 g (2.2 mmol) of potassium carbonate powder and 1.4 g (8.4 mmol) of potassium iodide were added. The reaction mixture was cooled to 0 °C and 5.0 g (33 mmol) of 4-(chloromethyl)-5-methyl-1,3-dioxol-2-one was added at 0 to 5°C. After the addition, the reaction mixture was warmed to 40-45 °C within one hour, then the mixture was stirred at this temperature for 2h. The sample of reaction mixture was analysed (HPLC; tritylolmesartan medoxomil 97.22 %, 4-(2-hydroxypropan-2-yl)-2-propyl-1-((2'-(1-trityl-1H-tetrazol-5-yl)biphenyl-4-yl)methyl)-1H-imidazole-5-carboxylate 0.09 %). The mixture was cooled to 10 to 20 °C and then 250 ml of ethyl acetate was added. The mixture was cooled again to 5-10 °C and then 200 ml of 10 % NaCl was added slowly. The temperature should not be higher than 25 °C during the addition. The phases were mixed separated and organic phase was washed with 100 ml of 10 % NaCl (2×) and dried over anhydrous sodium sulphate. After the filtration filtrate was evaporated under reduced pressure at temperature under 45°C to oily residue. To the residue 30 ml of acetonitrile was added at temperature not more than 45°C. The mixture was stirred at this temperature for 10 minutes then was cooled to 20 to 25°C and stirred at this temperature for 0.5 h and after that 3h at 0 to 5°C. The suspension was filtered, the cake washed with cold acetonitrile and dried at 40 to 50°C.

Yield: 17.0 g (94%)
HPLC: 99.72 % of the product, all impurities are under 0.1%.
IR: 3408, 1818, 1805, 1741, 1681, 1529, 1147, 1003, 699
XRD: Figure 1

### Example 2: Tritylolmesartan medoxomil ((5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate) from potassium 4-(2-hydroxypropan-2-yl)-2-propyl-1-((2'-(1-trityl-1H-tetrazol-5-yl)biphenyl-4-yl)methyl)-1H-imidazole-5-carboxylate

In 130 ml of *N*,*N*-dimethyl acetamide, 14.5 g (20 mmol) of potassium 4-(2-hydroxypropan-2-yl)-2-propyl-1-((2'-(1-trityl-1H-tetrazol-5-yl)biphenyl-4-yl)methyl)-1H-imidazole-5-carboxylate, 3.0 g (2.2 mmol) of potassium carbonate powder and 1.4 g (8.4 mmol) of potassium iodide were added. The mixture was cooled to 0 °C and 5.0 g (33 mmol) of 4-(chloromethyl)-5-methyl-1,3-dioxol-2-one was added at 0 to 5°C. After the addition, the reaction mixture was warmed to 40-45 °C within one hour, then the mixture was stirred at this temperature for 2h. The sample of reaction mixture was analysed (HPLC; tritylolmesartan medoxomil, 97.44%, 4-(2-hydroxypropan-2-yl)-2-propyl-1-((2'-(1-trityl-1H-tetrazol-5-yl)biphenyl-4-yl)methyl)-1H-imidazole-5-carboxylate 0.06 %). The mixture was cooled to 10 to 20°C and then 250 ml of ethyl acetate was added. The mixture was cooled again to 5-10 °C and then 200 ml of 10% NaCl was added slowly. The temperature should not be higher than 25 °C during the addition. The phases were mixed separated and organic phase was washed with 100 ml of 10% NaCl (2×) and dried over anhydrous sodium sulphate. After the filtration filtrate was evaporated under reduced pressure at temperature under 45°C to oily residue. To the residue 30 ml of acetonitrile was added at temperature not more than 45°C. The mixture was stirred at this temperature for 10 minutes then was cooled to 20 to 25°C and stirred at this temperature for 0.5 h and after that 3h at 0 to 5°C. The suspension was filtered, washed with cold acetonitrile and dried at 40 to 50°C.

Yield: 17.0 g (91%)
HPLC: 99.64 % of the product, all other impurities under 0.1%.
IR: 3408, 1818, 1805, 1741, 1681, 1529, 1148, 1002, 699

OMEK: 4-(2-hydroxypropan-2-yl)-2-propyl-1-((2'-(1-trityl-1H-tetrazol-5-yl)biphenyl-4-yl)methyl)-1 H-imidazole-5-carboxylate

### Example 3: Olmesartan medoxomil (5-methyl-2-oxo-1,3-dioxol-4-yl)methyl1-((2'-(1H-tetrazol-5-yl)biphenyl-4-yl)methyl)-4-(2-hydroxypropan-2-yl)-2-propyl-1H-imidazole-5-carboxylate

To 12.0g (15 mmol) of tritylolmesartan medoxomil prepared according to examples 1 or 2 69 ml of ethyl acetate was added and after that 12 ml of methanol and 2.67 ml (32 mmol) of conc. HCl were added. The mixture was stirred at room temperature for 2h and then the mixture was cooled to below 2°C. To the cooled mixture 71 ml of 1% NH₃ was slowly added to adjust pH to 4.93. The phases were separated and water phase was reextracted with 20 ml of ethyl acetate. Collected organic phases were dried over Na₂SO₄, filtered and concentrated under reduced pressure to approximately 15g of oily residue. To this residue 15 ml of ethyl acetate were added and the mixture was cooled to 20 °C and stirred at this temperature for 2h, and then cooled under 0°C and stirred at this temperature for 1hour. The product was filtered off and washed with 5 ml of fresh ethyl acetate and dried.

Yield: 7.55 g (90%).
HPLC: 99.81 % of the product, all individual impurities under 0.10%.

### Crystallization olmesartan medoxomil from tetrahydrofuran

10 g of olmesartan medoxomil was dissolved in 43 ml of THF at reflux temperature. The solution was filtered and filtrate was cooled to room temperature and stirred at this temperature for 1h. During this process olmesartan medoxomil was slowly crystallized. The mixture was then cooled to 0°C and stirred at this temperature for 2.5 h. The product was collected and dried.

Yield. 5.65g
Melting point of the product: 182-184°C
IR: 1831, 1707, 1391, 1300, 1167, 1140, 1003, 7682, 765

### Example 4

100 g of olmesartan medoxomil, 62.5 g of hydrochlorothiazide, 260 g of microcrystalline cellulose, 512.5 g of lactose monohydrate and 100 g of low-substituted hydroxypropylcellulose were homogenised. Finally, 15 g of magnesium stearate was admixed to prepare compressing mixture. Loss on drying of the compressing mixture was 1.51 % w/w (performed using Mettler Toledo HR73 halogen moisture analyzer at 105°C for 5 minutes). Compressing mixture was compressed to cores with theoretical weight 210 mg. The hardness of the cores was 76-91 N (average hardness of 20 cores was 83 N) and disintegration time was 20 seconds (performed in purified water at 37°C according to Ph.Eur.).
Cores were coated with water-based film coating suspension of a ready-to-make mixture, commercially available as Opadry 85F34353 II pink. The theoretical weight of the coated tablets was 218 mg.

### Example 5

120 g of olmesartan medoxomil, 75 g of hydrochlorothiazide, 390 g of microcrystalline cellulose, 742.5 g of lactose monohydrate and 150 g of low-substituted hydroxypropylcellulose were homogenised. Finally, 22.5 g of magnesium stearate was admixed to prepare compressing mixture. Loss on drying of the compressing mixture was 1.14% w/w. Compressing mixture was compressed to cores with theoretical weight 250 mg. The hardness of the cores was 72-94 N (average hardness of 20 cores was 82 N) and disintegration time was 15 seconds.
Cores were coated with water-based film coating suspension of a ready-to-make mixture, commercially available as Opadry 85F28751 II white. The theoretical weight of the coated tablets was 260 mg.

### Example 6

120 g of olmesartan medoxomil, 75 g of hydrochlorothiazide, 729 g of microcrystalline cellulose and 30 g of povidone were homogenised for 2 minutes at high-shear mixer. 510 g of purified water was sprayed onto the homogenised mixture and kneaded. The obtained granulate was dried in a fluid bed dryer at inlet air temeperature 57°C to the final temperature of the granulate 38°C for 30 minutes. The obtained granulate was sieved and loss on drying of the the compressing mixture of the granulate was 1.76% w/w. 75 g of croscarmellose sodium was admixed to the granulate. Finally, 21 g of magnesium stearate was admixed to prepare compressing mixture. Loss on drying of the compressing mixture was 1.67% w/w. Compressing mixture was compressed to cores with theoretical weight 250 mg. The hardness of the cores was 70-90 N (average hardness of 20 cores was 79 N) and disintegration time was 1 minute.
Cores were coated as described in Example 5.

### Example 7

120 g of olmesartan medoxomil, 75 g of hydrochlorothiazide, 1134 g of sorbitol and 150 g of low-substituted hydroxypropylcellulose were homogenised. Finally, 21 g of magnesium stearate was admixed to prepare compressing mixture. Loss on drying of the compressing mixture was 0.60% w/w. Compressing mixture was compressed to cores with theoretical weight 250 mg. The hardness of the cores was 65-90 N (average hardness of 20 cores was 75 N) and disintegration time was 5.5 minutes.
Cores were coated as described in Example 5.

### Example 8

120 g of olmesartan medoxomil, 150 g of hydrochlorothiazide, 390 g of microcrystalline cellulose, 667.5 g of lactose monohydrate and 150 g of low-substituted hydroxypropylcellulose were homogenised. Finally, 22.5 g of magnesium stearate was admixed to prepare compressing mixture. Loss on drying of the compressing mixture was 1.16% w/w. Compressing mixture was compressed to cores with theoretical weight 250 mg. The hardness of the cores was 106-128 N (average hardness of 20 cores was 116 N) and disintegration time was 15 seconds.
Cores were coated as described in Example 5.

### Example 9

120 g of olmesartan medoxomil, 37.5 g of hydrochlorothiazide, 390 g of microcrystalline cellulose, 780 g of lactose monohydrate and 150 g of low-substituted hydroxypropylcellulose were homogenised. Finally, 22.5 g of magnesium stearate was admixed to prepare compressing mixture. Loss on drying of the compressing mixture was 1.21% w/w. Compressing mixture was compressed to cores with theoretical weight 500 mg. The hardness of the cores was 126-145 N (average hardness of 20 cores was 135 N) and disintegration time was 15 seconds.
Cores were coated with water-based film coating suspension of a ready-to-make mixture, commercially available as Opadry 85F28751 II white. The theoretical weight of the coated tablets was 520 mg.

### Example 10

Compressing mixture, prepared as described in Example 2, was compressed to cores with theoretical weight 500 mg. The hardness of the cores was 146-188 N and disintegration time was 15-20 seconds.
Cores were coated as described in Example 9.

## Claims

1. A process for the preparation of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate comprising the steps of:
- esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid with a 4-halomethyl-2-oxo-1,3-dioxolene derivative in the presence of an inorganic base and an iodide and optionally in an organic solvent, wherein halo means Cl or Br,
- providing a solution of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate (tritylolmesartan medoxomil) in organic solvent selected from the group consisting of ethyl acetate, isopropyl acetate, propyl acetate, butyl acetate, isobutyl acetate, *tert*-butyl acetate; toluene, cyclohexane, hexane, heptane, diethyl ether, *tert-*butylmethyl ether and mixtures thereof,
- optionally concentrating the solution, and
- addition of another solvent selected from group consisting of acetone, acetonitrile, isopropanol and ethanol and
- isolating solid (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate.

2. The process according to claim 1 wherein the esterification comprises:
- providing the solution of 4-(2-hydroxypropan-2-yl)-2-propyl-1-((2'-(1-trityl-1H-tetrazol-5-yl)biphenyl-4-yl)methyl)-1H-imidazole-5-carboxylic acid in an organic solvent selected from N,N-dimethylformamide, N,N-dimethylacetamide, dimethyl sulfoxide, acetonitrile and any mixtures thereof, preferably dimethyl formamide and N,N-dimethylacetamide, more preferably N,N-dimethylacetamide,
- addition of an inorganic base such as hydroxides, in particular alkali or earth alkaline metal hydroxides such as KOH, NaOH, LiOH, carbonates, in particular alkali or earth alkaline metal carbonates such as Na₂CO₃, K₂CO₃, phosphates, in particular alkali or earth alkaline metal phosphates such as Na₃PO₄, preferably Na₂CO₃, K₂CO₃, more preferably K₂CO₃,
- addition of an iodide, in particular alkali or earth alkaline metal iodide that can be selected from the group consisting of KI, NaI, LiI, preferably KI,
- addition of 4-halomethyl-2-oxo-1,3-dioxolene derivative wherein halo means Cl or Br,
- performing esterification at elevated temperature as for example between 20 and 60°C for 1 to 24h, preferably for 1 to 5h.

3. The process according to any one of claims 1 to 2 wherein 4-(2-hydroxypropan-2-yl)-2-propyl-1-((2'-(1-trityl-1H-tetrazol-5-yl)biphenyl-4-yl)methyl)-1H-imidazole-5-carboxylic acid is prepared by the steps of:
- suspending of ethyl 4-(2-hydroxypropan-2-yl)-2-propyl-1-((2'-(1-trityl-1H-tetrazol-5-yl)biphenyl-4-yl)methyl)-1H-imidazole-5-carboxylate in an organic solvent selected from N,N-dimethylformamide, N,N-dimethylacetamide, dimethylsulfoxide, acetonitrile and any mixtures thereof, preferably dimethyl formamide and N,N-dimethylacetamide, more preferably N,N-dimethylacetamide,
- addition of an inorganic base such as KOH, NaOH, LiOH,
- performing the hydrolysis at room temperature for 1 to 24h, preferably for 1 to 5h.

4. A process for preparing (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate, which comprises a step of preparing (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1 H-imidazole-5-carboxylate according to any one of claims 1 to 3, followed by conversion of (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate to (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate.

5. The process according to claim 4 wherein the purity of (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate determined by HPLC is greater than 99.5, preferably greater than 99.8%.

6. The process according to claim 4, wherein pure solid (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate obtained by the process according to any one of claims 1 to 3 and having purity greater than 98%, preferably greater than 99%, the most preferably greater than 99.5 % with impurities less than 0.1% and wherein purity is preferably determined by HPLC is used for the manufacture of (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate.

7. The process according to claim 6, wherein the (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate is specified by an X-ray powder diffraction pattern exhibiting peaks at the following diffraction angles: 10.2 and 21.7 2Θ.

8. The process according to claim 7, wherein the (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate is specified by an X-ray powder diffraction pattern exhibiting peaks at the following diffraction angles: 3.0, 10.2, 12.1, 15.6, 18.4, 21.7 and 24.32Θ.

9. The process according to claim 6, wherein the (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate has an X-ray powder diffraction pattern substantially as shown in Figure 1.

10. The process according to any one of claims 6 to 8, comprising the use of pure solid (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate having purity greater than 98%, preferably greater than 99%, the most preferably greater than 99.5 % with impurities less than 0.1% and having an X-ray powder diffraction pattern exhibiting peaks at the diffraction angles: 3.0, 10.2, 12.1, 15.6, 18.4, 21.7 and 24.3 2Θ wherein said (5-methyl-2-oxo- ,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate is prepared by a process comprising the steps of:
- esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid with a 4-halomethyl-2-oxo-1,3-dioxolene derivative in the presence of an inorganic base and an iodide and optionally in an organic solvent, wherein halo means Cl or Br,
- isolating pure solid (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate as specified in claim 1, for preparing (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate.

11. A process for preparing (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate according to claim 1, wherein an iodide selected from the group consisting of KI, NaI, LiI, preferably KI, is added in the step of esterification of 4-(2-hydroxypropan-2-yl)-2-propyl-1-((2'-(1-trityl-1H-tetrazol-5-yl)biphenyl-4-yl)methyl)-1H-imidazole-5-carboxylic acid with a 4-halomethyl-2-oxo-1,3-dioxolene derivative, wherein halo means Cl or Br, wherein esterification is carried out in the presence of an inorganic base and wherein the isolation of the obtained compound is performed in an organic solvent selected from ethers such as diethyl ether, diisopropyl ether, tert-butylmethyl ether; esters such as ethyl acetate, isopropyl acetate, propyl acetate, butyl acetate, isobutyl acetate, tert-butyl acetate; acetonitrile, toluene, cyclohexane, hexane, heptane, alcohols such as methanol, ethanol, isopropanol; water and any mixtures thereof.

12. Process for preparing a pharmaceutical composition comprising (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl-1H-imidazole-5-carboxylate comprising the step of manufacturing (5-methyl-2-oxo-1,3-dioxol-4-yl)methyl 4-(2-hydroxypropan-2-yl)-2-propyl-1-((2'-(1-trityl-1H-tetrazol-5-yl)biphenyl-4-yl)methyl)-1H-imidazole-5-carboxylate as specified in any one of the present process claims 1 to 5, and 11.

13. The process according to claim 12 further comprising the incorporation of one or more other active substance and at least one pharmaceutically acceptable ingredient selected from the group consisting of binders, surfactants, diluents, disintegrants, lubricants and optionally coating layer into the pharmaceutical composition.

14. The process according to claim 13 wherein the active substances are (5-methyl-2-oxo-1,3-dioxol-4-yl)methyl 1-((2'-(1H-tetrazol-5-yl)biphenyl-4-yl)methyl)-4-(2-hydroxypropan-2-yl)-2-propyl- 1H-imidazole-5-carboxylate and hydrochlorothiazide.

## Patentansprüche

1. Verfahren zur Herstellung von (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat, umfassend die Schritte:
- Verestern von 4-(2-Hydroxypropan-2-yl)-2-propyl-1-((2'-(1-trityl-1H-tetrazol-5-yl)biphenyl-4-yl)methyl)-1H-imidazol-5-carbonsäure mit einem 4-Halogenmethyl-2-oxo-1,3-dioxolen-Derivat in Gegenwart einer anorganischen Base und eines Iodids und gegebenenfalls in einem organischen Lösungsmittel, wobei Halogen Cl oder Br bedeutet,
- Bereitstellen einer Lösung von (5-Methyl-2-oxo-1,3-dioxol-4-yl)methyl-4-(2-hydroxypropan-2-yl)-2-propyl-1-((2'-(1-trityl-1H-tetrazol-5-yl)-biphenyl-4-yl)methyl)-1H-imidazol-5-carboxylat (Tritylolmesartan-medoxomil) in einem organischen Lösungsmittel, ausgewählt aus der Gruppe bestehend aus Ethylacetat, Isopropylacetat, Propylacetat, Butylacetat, Isobutylacetat, tert-Butylacetat, Toluol, Cyclohexan, Hexan, Heptan, Diethylether, tert-Butylmethylether und Mischungen davon,
- gegebenenfalls Konzentrieren der Lösung und
- Zugabe eines anderen Lösungsmittels, ausgewählt aus der Gruppe bestehend aus Aceton, Acetonitril, Isopropanol und Ethanol, und
- Isolieren des festen (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylats.

2. Verfahren gemäss Anspruch 1, wobei die Veresterung umfasst:
- Bereitstellen einer Lösung von 4-(2-Hydroxy-propan-2-yl)-2-propyl-1-((2'-(1-trityl-1H-tetrazol-5-yl)biphenyl-4-yl)methyl)-1H-imidazol-5-carbonsäure in einem organischen Lösungsmittel, ausgewählt aus N,N-Dimethylformamid, N,N-Dimethylacetamid, Dimethylsulfoxid, Acetonitril und jeglichen Mischungen davon, vorzugsweise Dimethylformamid und N,N-Dimethylacetamid, stärker bevorzugt N,N-Dimethylacetamid,
- Zugabe einer anorganischen Base, wie Hydroxiden, insbesondere Alkali- oder Erdalkalimetallhydroxiden, wie KOH, NaOH, LiOH, Carbonaten, insbesondere Alkali- oder Erdalkalimetallcarbonaten, wie Na₂CO₃, K₂CO₃, Phosphaten, insbesondere Alkali- oder Erdalkalimetallphosphaten, wie Na₃PO₄, vorzugsweise Na₂CO₃, K₂CO₃, stärker bevorzugt K₂CO₃,
- Zugabe eines Iodids, insbesondere Alkali- oder Erdalkalimetalliodid, das aus der Gruppe bestehend aus KI, NaI, LiI, vorzugsweise KI, ausgewählt werden kann,
- Zugabe eines 4-Halogenmethyl-2-oxo-1,3-dioxolen-Derivats, wobei Halogen Cl oder Br bedeutet,
- Durchführen einer Veresterung bei erhöhter Temperatur, beispielsweise zwischen 20 und 60°C, während 1 bis 24 Stunden, vorzugsweise 1 bis 5 Stunden.

3. Verfahren gemäss irgendeinem der Ansprüche 1 bis 2, wobei die 4-(2-Hydroxypropan-2-yl)-2-propyl-1-((2'-(1-trityl-1H-tetrazol-5-yl)biphenyl-4-yl)methyl)-1H-imidazol-5-carbonsäure hergestellt wird durch die Schritte:
- Suspendieren von Ethyl-4-(2-hydroxypropan-2-yl)-2-propyl-1-((2'-(1-trityl-1H-tetrazol-5-yl)biphenyl-4-yl)methyl)-1H-imidazol-5-carboxylat in einem organischen Lösungsmittel, ausgewählt aus N,N-Dimethylformamid, N,N-Dimethylacetamid, Dimethylsulfoxid, Acetonitril und jeglichen Mischungen davon, vorzugsweise Dimethylformamid und N,N-Dimethylacetamid, stärker bevorzugt N,N-Dimethylacetamid,
- Zugabe einer anorganischen Base, wie KOH, NaOH und LiOH,
- Durchführen der Hydrolyse bei Raumtemperatur während 1 bis 24 Stunden, vorzugsweise 1 bis 5 Stunden.

4. Verfahren zur Herstellung von (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat, umfassend einen Schritt zur Herstellung von (5-Methyl-2-oxo-1,3-dioxol-4-yl)-methyl-4-(2-hydroxypropan-2-yl)-2-propyl-1-((2'-(1-trityl-1H-tetrazol-5-yl)biphenyl-4-yl)methyl)-1H-imidazol-5-carboxylat gemäss irgendeinem der Ansprüche 1 bis 3, gefolgt von Umwandeln von (5-Methyl-2-oxo-1,3-dioxol-4-yl)methyl-4-(2-hydroxy-propan-2-yl)-2-propyl-1-((2'-(1-trityl-1H-tetrazol-5-yl)biphenyl-4-yl)methyl)-1H-imidazol-5-carboxylat zu (5-Methyl-2-oxo-1,3-dioxol-4-yl)methyl-1-((2'-(1H-tetrazol-5-yl)biphenyl-4-yl)methyl)-4-(2-hydroxy-propan-2-yl)-2-propyl-1H-imidazol-5-carboxylat.

5. Verfahren gemäss Anspruch 4, wobei die Reinheit von (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat, bestimmt durch HPLC, grösser als 99,5 %, vorzugsweise grösser als 99,8 % ist.

6. Verfahren gemäss Anspruch 4, wobei reines festes (5-Methyl-2-oxo-1,3-dioxol-4-yl)methyl-4-(2-hydroxy-propan-2-yl)-2-propyl-1-((2'-(1-trityl-1H-tetrazol-5-yl)biphenyl-4-yl)methyl)-1H-imidazol-5-carboxylat, das nach dem Verfahren gemäss irgendeinem der Ansprüche 1 bis 3 erhalten wurde und eine Reinheit von grösser als 98 %, vorzugsweise grösser als 99 %, am meisten bevorzugt grösser als 99,5 % mit Verunreinigungen von weniger als 0,1 % aufweist und wobei die Reinheit vorzugsweise durch HPLC bestimmt ist, zur Herstellung von (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat verwendet wird.

7. Verfahren gemäss Anspruch 6, wobei das (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat durch Pulver-Röntgendiffraktionsmuster gekennzeichnet ist, die Peaks bei den nachstehenden Diffraktionswinkeln zeigen: 10,2 und 21,7 2θ.

8. Verfahren gemäss Anspruch 7, wobei das (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat durch Pulver-Röntgendiffraktionsmuster gekennzeichnet ist, die Peaks bei den nachstehenden Diffraktionswinkeln zeigen: 3,0, 10,2, 12,1, 15,6, 18,4, 21,7 und 24,3 2θ.

9. Verfahren gemäss Anspruch 6, wobei das (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat im wesentlichen das in Fig. 1 gezeigte Pulver-Röntgendiffraktionsmuster aufweist.

10. Verfahren gemäss irgendeinem der Ansprüche 6 bis 8, umfassend die Verwendung von reinem festen (5-Methyl-2-oxo-1,3-dioxol-4-yl)methyl-4-(2-hydroxypropan-2-yl)-2-propyl-1-((2'-(1-trityl-1H-tetrazol-5-yl)-biphenyl-4-yl)methyl)-1H-imidazol-5-carboxylat mit einer Reinheit von grösser als 98 %, vorzugsweise grösser als 99 %, am meisten bevorzugt grösser als 99,5 %, mit Verunreinigungen von weniger als 0,1 % und mit Pulver-Röntgendiffraktionsmustern, die Peaks bei den nachstehenden Diffraktionswinkeln zeigen: 3,0, 10,2, 12,1, 15,6, 18,4, 21,7 und 24,3 2θ, wobei das (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat nach einem Verfahren hergestellt wird, umfassend die Schritte:
- Verestern von 4-(2-Hydroxypropan-2-yl)-2-propyl-1-((2'-(1-trityl-1H-tetrazol-5-yl)biphenyl-4-yl)methyl)-1H-imidazol-5-carbonsäure mit einem 4-Halogenmethyl-2-oxo-1,3-dioxolen-Derivat in Gegenwart einer anorganischen Base und eines Iodids und gegebenenfalls in einem organischen Lösungsmittel, wobei Halogen Cl oder Br bedeutet,
- Isolieren von reinem festen (5-Methyl-2-oxo-1,3-dioxol-4-yl)methyl-4-(2-hydroxypropan-2-yl)-2-propyl-1-((2'-(1-trityl-1H-tetrazol-5-yl)-biphenyl-4-yl)methyl)-1H-imidazol-5-carboxylat, wie in Anspruch 1 angegeben, zur Herstellung von (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat.

11. Verfahren zur Herstellung von (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)-methyl)-1H-imidazol-5-carboxylat gemäss Anspruch 1, wobei ein Iodid, ausgewählt aus der Gruppe bestehend aus KI, NaI, LiI, vorzugsweise KI, in dem Schritt der Veresterung von 4-(2-Hydroxypropan-2-yl)-2-propyl-1-((2'-(1-trityl-1H-tetrazol-5-yl)biphenyl-4-yl)methyl)-1H-imidazol-5-carbonsäure mit einem 4-Halogenmethyl-2-oxo-1,3-dioxolen-Derivat, wobei Halogen Cl oder Br bedeutet, zugegeben wird, wobei die Veresterung in Gegenwart einer anorganischen Base durchgeführt wird und wobei die Isolierung der erhaltenen Verbindung in einem organischen Lösungsmittel, ausgewählt aus Ethern, wie Diethylether, Diisopropylether, tert-Butylmethylether; Estern, wie Ethylacetat, Isopropylacetat, Propylacetat, Butylacetat, Isobutylacetat, tert-Butylacetat; Acetonitril, Toluol, Cyclohexan, Hexan, Heptan, Alkoholen, wie Methanol, Ethanol, Isopropanol; Wasser und jeglichen Mischungen davon durchgeführt wird.

12. Verfahren zur Herstellung einer pharmazeutischen Zusammensetzung, umfassend (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat, das den Schritt der Herstellung von (5-Methyl-2-oxo-1,3-dioxol-4-yl)methyl-4-(2-hydroxypropan-2-yl)-2-propyl-1-((2'-(1-trityl-1H-tetrazol-5-yl)biphenyl-4-yl)methyl)-1H-imidazol-5-carboxylat, wie in irgendeinem der vorstehenden Verfahrensansprüche 1 bis 5 und 11 angegeben, umfasst.

13. Verfahren gemäss Anspruch 12, das ferner das Einfügen von einer oder mehreren anderen aktiven Substanz(en) und mindestens eines pharmazeutisch annehmbaren Bestandteils, ausgewählt aus der Gruppe bestehend aus Bindemitteln, Tensiden, Verdünnungsmitteln, Sprengmitteln, Gleitmitteln und gegebenenfalls Beschichtungsschichten, in die pharmazeutische Zusammensetzung umfasst.

14. Verfahren gemäss Anspruch 13, wobei die aktiven Substanzen (5-Methyl-2-oxo-1,3-dioxol-4-yl)methyl-1-((2'-(1H-tetrazol-5-yl)biphenyl-4-yl)methyl)-4-(2-hydroxypropan-2-yl)-2-propyl-1H-imidazol-5-carboxylat und Hydrochlorthiazid sind.

## Revendications

1. Procédé pour préparer du 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle, comprenant les étapes suivantes :
- estérification d'acide 4-(2-hydroxypropan-2-yl)-2-propyl-1-((2'-(1-trityl-1H-tétrazol-5-yl)biphényl-4-yl)-méthyl)-1H-imidazole-5-carboxylique avec un dérivé de 4-halogénométhyl-2-oxo-1,3-dioxolène en présence d'une base inorganique et d'un iodure et éventuellement dans un solvant organique, halogéno signifiant Cl ou Br,
- obtention d'une solution de 4-(2-hydroxypropan-2-yl)-2-propyl-1-((2'-(1-trityl-1H-tétrazol-5-yl)biphényl-4-yl)-méthyl)-1H-imidazole-5-carboxylate de (5-méthyl-2-oxo-1,3-dioxol-4-yl)méthyle (tritylolmésartan médoxomil) dans un solvant organique choisi dans le groupe constitué par l'acétate d'éthyle, l'acétate d'isopropyle, l'acétate de propyle, l'acétate de butyle, l'acétate d'isobutyle, l'acétate de tert-butyle, le toluène, le cylohexane, l'hexane, l'heptane, le diéthyléther, le tert-butylméthyléther et leurs mélanges,
- éventuellement concentration de la solution, et
- addition d'un autre solvant choisi dans le groupe constitué par l'acétone, l'acétonitrile, l'isopropanol et l'éthanol, et
- isolation de 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle solide.

2. Procédé selon la revendication 1, dans lequel l'estérification comprend :
- l'obtention de la solution d'acide 4-(2-hydroxypropan-2-yl)-2-propyl-1-((2'-(1-trityl-1H-tétrazol-5-yl)biphényl-4-yl)méthyl)-1H-imidazole-5-carboxylique dans un solvant organique choisi parmi le N,N-diméthylformamide, le N,N-diméthylacétamide, le diméthylsulfoxyde, l'acétonitrile et l'un quelconque de leurs mélanges, de préférence le diméthylformamide et le N,N-diméthylacétamide, mieux encore le N,N-diméthylacétamide,
- l'addition d'une base inorganique telle qu'un hydroxyde, en particulier un hydroxyde de métal alcalin ou alcalino-terreux tel que KOH, NaOH, LiOH, un carbonate, en particulier un carbonate de métal alcalin ou alcalino-terreux tel que Na₂CO₃, K₂CO₃, un phosphate, en particulier un phosphate de métal alcalin ou alcalino-terreux tel que Na₃PO₄, de préférence Na₂CO₃, K₂CO₃, mieux encore K₂CO₃,
- l'addition d'un iodure, en particulier d'un iodure de métal alcalin ou alcalino-terreux qui peut être choisi dans le groupe constitué par KI, NaI, LiI, de préférence KI,
- l'addition d'un dérivé de 4-halogénométhyl-2-oxo-1,3-dioxolène, halogéno signifiant Cl ou Br,
- la mise en oeuvre d'une estérification à une température élevée, comprise par exemple entre 20 et 60°C, pendant 1 à 24 heures, de préférence pendant 1 à 5 heures.

3. Procédé selon l'une quelconque des revendications 1 et 2, dans lequel l'acide 4-(2-hydroxypropan-2-yl)-2-propyl-1-((2'-(1-trityl-1H-tétrazol-5-yl)biphényl-4-yl)méthyl)-1H-imidazole-5-carboxylique est préparé par les étapes de :
- mise en suspension de 4-(2-hydroxypropan-2-yl)-2-propyl-1-((2'-(1-trityl-1H-tétrazol-5-yl)biphényl-4-yl)méthyl)-1H-imidazole-5-carboxylate d'éthyle dans un solvant organique choisi parmi le N,N-diméthylformamide, le N,N-diméthylacétamide, le diméthylsulfoxyde, l'acétonitrile et l'un quelconque de leurs mélanges, de préférence le diméthylformamide et le N,N-diméthylacétamide, mieux encore le N,N-diméthylacétamide,
- addition d'une base inorganique telle que KOH, NaOH, LiOH,
- mise en oeuvre de l'hydrolyse à la température ambiante pendant 1 à 24 heures, de préférence pendant 1 à 5 heures.

4. Procédé pour préparer du 1-((2'-(1H-tétrazol-5-yl)biphényl-4-yl)méthyl)-4-(2-hydroxypropan-2-yl)-2-propyl-1H-imidazole-5-carboxylate de (5-méthyl-2-oxo-1,3-dioxol-4-yl)méthyle, comprenant une étape de préparation de 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle selon l'une quelconque des revendications 1 à 3, suivie de la conversion du 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle en 1-((2'-(1H-tétrazol-5-yl)biphényl-4-yl)méthyl)-4-(2-hydroxypropan-2-yl)-2-propyl-1H-imidazole-5-carboxylate de (5-méthyl-2-oxo-1,3-dioxol-4-yl)méthyle.

5. Procédé selon la revendication 4, dans lequel la pureté du 1-((2'-(1H-tétrazol-5-yl)biphényl-4-yl)méthyl)-4-(2-hydroxypropan-2-yl)-2-propyl-1H-imidazole-5-carboxylate de (5-méthyl-2-oxo-1,3-dioxol-4-yl)méthyle, déterminée par CLHP, est supérieure à 99,5 %, de préférence supérieure à 99,8 %.

6. Procédé selon la revendication 4, dans lequel du 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle solide pur, obtenu par le procédé selon l'une quelconque des revendications 1 à 3 et ayant une pureté supérieure à 98 %, de préférence supérieure à 99 %, tout spécialement supérieure à 99,5 % avec moins de 0,1 % d'impuretés, la pureté étant de préférence déterminée par CLHP, est utilisé pour la fabrication de 1-((2'-(1H-tétrazol-5-yl)-biphényl-4-yl)méthyl)-4-(2-hydroxypropan-2-yl)-2-propyl-1H-imidazole-5-carboxylate de (5-méthyl-2-oxo-1,3-dioxol-4-yl)méthyle.

7. Procédé selon la revendication 6, dans lequel le 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle est spécifié par un motif de diffraction des rayons X par la technique des poudres présentant des pics aux angles de diffraction suivants : 10,2 et 21,7 20.

8. Procédé selon la revendication 7, dans lequel le 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle est spécifié par un motif de diffraction des rayons X par la technique des poudres présentant des pics aux angles de diffraction suivants : 3,0, 10,2, 12,1, 15,6, 18,4, 21,7 et 24,3 2Θ.

9. Procédé selon la revendication 6, dans lequel le 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle a un motif de diffraction des rayons X par la technique des poudres pratiquement tel que représenté sur la Figure 1.

10. Procédé selon l'une quelconque des revendications 6 à 8, comprenant l'utilisation de 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle solide pur ayant une pureté supérieure à 98 %, de préférence supérieure à 99 %, tout spécialement supérieure à 99,5 % avec moins de 0,1 % d'impuretés, et ayant un motif de diffraction des rayons X par la technique des poudres présentant des pics aux angles de diffraction suivants : 3,0, 10,2, 12,1, 15,6, 18,4, 21,7 et 24,3 2Θ, dans lequel ledit 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle est préparé par un procédé comprenant les étapes de :
- estérification d'acide 4-(2-hydroxypropan-2-yl)-2-propyl-1-((2'-(1-trityl-1H-tétrazol-5-yl)biphényl-4-yl)méthyl)-1H-imidazole-5-carboxylique avec un dérivé de 4-halogénométhyl-2-oxo-1,3-dioxolène en présence d'une base inorganique et d'un iodure et éventuellement dans un solvant organique, halogéno signifiant Cl ou Br,
- isolation de 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle solide pur comme spécifié dans la revendication 1,
pour la préparation de 1-((2'-(1H-tétrazol-5-yl)biphényl-4-yl)méthyl)-4-(2-hydroxypropan-2-yl)-2-propyl-1H-imidazole-5-carboxylate de (5-méthyl-2-oxo-1,3-dioxol-4-yl)méthyle.

11. Procédé pour préparer du 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle selon la revendication 1, dans lequel un iodure choisi dans le groupe constitué par KI, NaI, LiI, de préférence KI, est ajouté dans l'étape d'estérification d'acide 4-(2-hydroxypropan-2-yl)-2-propyl-1-((2'-(1-trityl-1H-tétrazol-5-yl)biphényl-4-yl)-méthyl)-1H-imidazole-5-carboxylique avec un dérivé de 4-halogénométhyl-2-oxo-1,3-dioxolène, halogéno signifiant Cl ou Br, dans lequel l'estérification est mise en oeuvre en présence d'une base inorganique, et dans lequel l'isolation du composé obtenu est effectuée dans un solvant organique choisi parmi les éthers tels que le diéthyléther, le diisopropyléther, le tert-butylméthyléther ; les esters tels que l'acétate d'éthyle, l'acétate d'isopropyle, l'acétate de propyle, l'acétate de butyle, l'acétate d'isobutyle, l'acétate de tert-butyle ; l'acétonitrile, le toluène, le cyclohexane, l'hexane, l'heptane, les alcools tels que le méthanol, l'éthanol, l'isopropanol ; l'eau ; et l'un quelconque de leurs mélanges.

12. Procédé pour préparer une composition pharmaceutique comprenant du 1-((2'-(1H-tétrazol-5-yl)biphényl-4-yl)méthyl)-4-(2-hydroxypropan-2-yl)-2-propyl-1H-imidazole-5-carboxylate de (5-méthyl-2-oxo-1,3-dioxol-4-yl)méthyle, comprenant l'étape de fabrication de 4-(2-hydroxypropan-2-yl)-2-propyl-1-((2'-(1-trityl-1H-tétrazol-5-yl)biphényl-4-yl)méthyl)-1H-imidazole-5-carboxylate de (5-méthyl-2-oxo-1,3-dioxol-4-yl)méthyle comme spécifié dans l'une quelconque des présentes revendications de procédé 1 à 5 et 11.

13. Procédé selon la revendication 12, comprenant en outre l'incorporation d'une ou plusieurs autres substances actives et d'au moins un ingrédient pharmaceutiquement acceptable choisi dans le groupe constitué par les liants, les tensioactifs, les diluants, les délitants, les lubrifiants, et éventuellement d'une couche de revêtement, dans la composition pharmaceutique.

14. Procédé selon la revendication 13, dans lequel les substances actives sont le 1-((2'-(1H-tétrazol-5-yl)-biphényl-4-yl)méthyl)-4-(2-hydroxypropan-2-yl)-2-propyl-1H-imidazole-5-carboxylate de (5-méthyl-2-oxo-1,3-dioxol-4-yl)méthyle et l'hydrochlorothiazide.
